(19) Europäisches Patentamt
European Patent Office
Office européen des brevets

(11) **EP 4 400 040 A1**

(12) **EUROPEAN PATENT APPLICATION**

(43) Date of publication:
**17.07.2024 Bulletin 2024/29**

(21) Application number: **23151043.9**

(22) Date of filing: **11.01.2023**

(51) International Patent Classification (IPC):
**A61B 5/022** (2006.01)    **A61B 5/00** (2006.01)

(52) Cooperative Patent Classification (CPC):
**A61B 5/02225; A61B 5/7246**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **MUEHLSTEFF, Jens**
  **Eindhoven (NL)**

• **SCHMITT, Lars**
  **Eindhoven (NL)**
• **DAVIDOIU, Valentina-Geta**
  **5656AG Eindhoven (NL)**
• **WIJSHOFF, Ralph Wilhelm Christianus Gemma Rosa**
  **Eindhoven (NL)**

(74) Representative: **Philips Intellectual Property & Standards**
**High Tech Campus 52**
**5656 AG Eindhoven (NL)**

(54) **PULSE WAVE SIGNAL ACQUISITION**

(57) A method for deriving a pulse wave signal for a patient based on a a tissue pressure signal measured at an artery while an external pressure is being applied to the artery of a defined pressure value or range, and optionally wherein the defined pressure value or range is determined dynamically based on characteristics of the tissue pressure waveform.

FIG. 13

**Description**

FIELD OF THE INVENTION

The present invention relates to a method for measuring a pulse waveform of a patient.

BACKGROUND OF THE INVENTION

Monitoring of hemodynamic parameters plays an important role in patient monitoring, particularly for critically ill patients. Hemodynamic monitoring allows for monitoring tissue perfusion and oxygen delivery while maintaining adequate mean arterial blood pressure for a patient. In most clinical practice, such monitoring is only available in more intensive care environments due to the invasive nature of currently available technologies.

In recent years, advances have been made in developing non-invasive hemodynamic monitoring techniques.

One area of interest is the possibility to use measurement data acquired using an inflatable measurement cuff (i.e. a sphygmomanometer system), typically used for blood pressure measurement, to also non-invasively determine estimates for central hemodynamic parameters such as cardiac output (CO), stroke volume (SV), stroke volume variation (SVV), in addition to various measures of arterial blood pressure, such as mean arterial pressure (MAP), systolic blood pressure (SBP) and diastolic blood pressure (DBP).

One example system developed for non-invasive hemodynamic monitoring is the device detailed in document EP2953528 A1.

This describes a blood pressure measuring system which includes a separate sensor for measuring a pressure between the user's body part (e.g. arm) and the cuff. This will be referred to as a tissue pressure sensor. This is provided in addition to a sensor for sensing a pressure inside the inflatable cuff. The tissue pressure sensor allows for obtaining a quasi-continuous pressure signal which, under certain circumstances, approximates an invasively acquired pulse waveform signal for the patient. This allows various hemodynamic parameters to be derived.

Document US 2015/0320364 A1 describes one example algorithm which enables converting an acquired pressure signal to an approximation of a pulse waveform signal. It is of general advantage to be able to non-invasively measure a patient's pulse waveform, preferably continuously, to avoid using an invasive arterial line. Previous methods in the art have only been able to acquire peripheral (not central) pulse waveform measurements, and there are accuracy issues. For example, traditional known methods include the volume clamp method, which uses a finger cuff, and controls the finger cuff to maintain a constant transmural pressure, so that a constant volume of blood is maintained in the artery.

It was proposed in US 2015/0320364 A1 to use instead measurements obtained using a pressure sensor in a blood pressure measurement cuff.

As a general rule however, it is not possible to simply use the directly measured pressure oscillation signal acquired from the readout of the pressure sensor of the blood pressure cuff. This is because, as a general rule, the relationship between a measured pulse signal (as a function of time) and a true arterial pulse signal (as a function of time) is non-linear with pressure - with the result that the measured pulse signal shape, in general, cannot be expected to match the shape of the arterial pulse wave.

This is thought to be due to the artery wall compliance affecting the linearity of pressure transfer between the arterial pressure and the measured pressure at the sensor.

However, it was realized that, notwithstanding the above general rule, there does exist an applied clamping pressure value where the relationship between the measured pulse signal as a function of time and the true arterial pressure is substantially linear, and thus where the pulse signal shape is more reliably reflective of an arterial pulse waveform shape.

This value was thought to be where the applied clamping pressure equals the mean arterial pressure, because at this clamping pressure the biasing/stress in the arterial wall is minimal, for example substantially 0.

The concept in US 2015/0320364 A1 is to compute a weighted average of a series of pulse wave signals acquired at different respective clamping pressures across a range, and wherein the weightings are related to, or correlated with, the proximity in pressure of a given signal's clamping pressure value to some characteristic clamping pressure which is defined as the clamping pressure for which the internal actual mean arterial pressure equals a certain percentage of the applied clamping pressure.

This characteristic pressure value however cannot be determined, so instead US 2015/0320364 A1 proposes an iterative method for determining the weightings.

Scaling was applied after the general shape of the pulse waveform had been determined using the weighted average signal. The scaling was based on measured values of SBP and DPB, and wherein the pulse waveform shape was scaled in amplitude so that its maximum value matched the SBP and its minimum value matched the DBP.

As a further enhancement of this method, in order to obtain a continuous central blood pressure waveform, rather than just intermittent discrete measures, an additional auxiliary continuous blood pressure measurement device was used (such as the volume clamp finger method), and wherein the continuous waveform output from this auxiliary sensor was adjusted or calibrated according to the intermittent pulse waveform measurements obtained using the more complex method already described above.

However, the algorithm in US 2015/0320364 A1 is very complex.

Thus, a simplified approach to deriving a pulse wave signal and/or for deriving hemodynamic parameters using a continuous pressure signal would be desirable.

SUMMARY OF THE INVENTION

The invention is defined by the claims.

According to examples in accordance with an aspect of the invention, there is provided a method for obtaining a pulse wave signal of a patient, the method employing use of a blood pressure measurement apparatus, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator.

The method comprises: acquiring a tissue pressure signal from the tissue pressure sensor at a defined applied pressure value or within a defined applied pressure range, and deriving a pulse wave signal from the tissue pressure signal based on extracting an AC component of the tissue pressure signal.

In some embodiments, (a) the defined applied pressure range is a range between 0 and 50 mmHg (= 0 to 6666 Pa), for example a range between 0 and 40 mmHg (= 0 to 5332 Pa).

In some embodiments, (b) the defined applied pressure value is equal to a pre-measured mean arterial pressure (MAP) value for the patient.

In some embodiments, (c) the defined applied pressure range is a range defined according to a characteristic feature or property of the tissue pressure signal which occurs in coincidence with the range, wherein the characteristic property or feature is occurrence of a maximum amplitude of the AC component of the tissue pressure signal.

Optionally, the method may further comprise generating a data output representative of the pulse wave signal. This provides an output representative of the patient's pulse waveform. This may be exported to an external device. For example, it may be communicated to a user interface for display to a user.

The tissue pressure signal is preferably acquired over at least one heart cycle.

The inventors have recognized that the measured pulse wave signal morphology will change as a function of applied cuff pressure. This is due to the relationship between transmural pressure and arterial wall compliance. Through experimentation and simulation, the inventors have found the interesting result that there are reliable and predictable ranges of applied external pressure within which a measured tissue pressure signal (AC component) will closely match the waveform morphology of the patient's true pulse waveform, due to these ranges being associated with approximately invariant arterial wall compliance.

Thus, the inventors have recognized that this fact can be used to obtain a continuous readout of a patient's pulse wave by applying an external pressure within one of the defined ranges or values, and optionally holding the external pressure within one of the defined ranges to enable a continuous pulse wave signal to be derived.

The particular defined pressure value or range for which the pulse wave signal is acquired might be defined quantitatively in advance, or it might be determined dynamically during the measurement process.

The method may comprise a step of obtaining the defined pressure value or range. The defined pressure value or range may be obtained from a datastore or a memory or a local register, e.g. in the event that the pressure value or range is pre-defined. The defined pressure value or range may be determined as part of the method.

For example, in some embodiments, the defined applied pressure value or range within which the reference tissue pressure signal is acquired is a range between 0-50 mmHg, for example between 0-40 mmHg (=0 to 5332 Pa), for example 10-40 mmHg (=1333 to 5332 Pa). As will be explained in detail later, through experimentation and simulation, it has been found by the inventors that a tissue pressure signal acquired at this particular range closely approximates the true pulse waveform for the patient. The value should be greater than zero and less than the defined upper limit for the range.

To increase SNR of the obtained pulse wave signal, in some embodiments, acquiring the tissue pressure signal comprises sampling a tissue pressure signal over a plurality of heart cycles at a constant applied pressure value, and computing an average tissue pressure signal over the multiple heart cycles as the tissue pressure signal. Optionally, before averaging, the amplitude and the period of each pulse may be normalized in order to obtain a cleaner averaged pulse.

In some embodiments, the defined pressure value or range is set according to an obtained measure of a physiological parameter of the patient. In particular this may be a value of a blood pressure of the patient.

In one particular set of embodiments, the defined pressure value or range is set to be a value at or around a value of the patient's mean arterial pressure (MAP). As will be explained in detail below, through simulation and experimentation, the inventors have found the non-obvious result that when the applied pressure matches the patient's mean arterial pressure, the disparity between the waveform morphology of an acquired tissue pressure signal and the patient's true pulse waveform is at a minimal level. In particular, this applied pressure level coincides with a local minimum in the disparity of the waveform with the true pulse waveform. Some small tolerance around the MAP value may be incorporated. For example, the pre-defined applied pressure value or range may be a range defined by MAP +/- 5% or MAP +/- 10%.

In some embodiments, instead of a fixed numerical value or range, the defined applied pressure value or range is defined according to a characteristic feature or property of the tissue pressure signal which occurs in coincidence with the applied pressure value or range. The characteristic feature or property may for example be the amplitude of the tissue pressure signal AC component being at or around a maximum. The method may therefore in practice comprise incrementally varying the applied pressure across a sequence of applied pressure values, monitoring the amplitude of the AC component of the real-time measured tissue pressure signal, and then detecting a maximum in this amplitude through known maximum detection techniques. The pulse wave signal can then be computed from the obtained tissue pressure signal which has the highest AC amplitude

When increasing the applied pressure, a maximum in the amplitude of the AC component is expected to occur in an applied pressure range around the mean arterial pressure (MAP). As will be explained below, from experiment and simulation it has been found that, at an applied pressure approximately equal to mean arterial pressure, the waveform disparity with respect to a true pulse wave for the patient is at or close to zero.

In some embodiments, the method comprises communicating with the blood pressure measurement apparatus to control the tissue applicator to apply said pressure at the defined applied pressure value within the defined applied pressure range, and to acquire the tissue pressure signal at the defined pressure value or within the defined applied pressure range. It may comprise holding the applied pressure constant at said pressure value or within said defined pressure range.

In some embodiments, the method comprises communicating with the blood pressure measurement apparatus to control a tissue application pressure cycle of the blood pressure measurement apparatus in which tissue application pressure is incrementally increased or decreased across an application pressure range to realize a sequence of different applied pressures which include said defined value or range of applied pressures. For example, this might be applied where embodiment (c) recited above is applied.

In some embodiments, the AC component of each acquired tissue pressure signal is determined in real time with the application pressure cycle, and wherein the application pressure cycle is terminated once a local maximum in the AC amplitude has been identified. This has the advantage of minimizing the time required for the measurement process since the process can simply be ended once the required pulse wave signal features have all been detected.

The application pressure cycle being terminated means for example that the pressure is relaxed back to zero applied pressure, or minimum applied pressure.

In some embodiments, the blood pressure measurement apparatus includes a cuff for wrapping around a part of the body of a user, and wherein the cuff incorporates the pressure applicator, and wherein the pressure applicator is a pneumatic actuator in the form of an inflatable bladder.

Here, the pressure application cycle comprises an inflation/deflation cycle of the cuff.

In some embodiments, deriving the pulse wave signal further comprises scaling the extracted AC component of the tissue pressure signal, wherein the scaling comprises, for each individual pulse in the signal, maintaining a waveform shape/morphology, but increasing the scale of the waveform such that a maximum point of the waveform matches a pre-measured SBP value, and the minimum point matches a pre-measured DBP value. Thus, scaling is performed. Scaling may additionally be performed in the time dimension such that the period spanned by each individual pulse of the pulse wave signal is scaled to a pre-defined period, for example 1 second. These operations together effectively achieve a normalization of each pulse in the signal in both the amplitude dimension and the time dimension. The phrase 'scaling and normalization' in this document may refer to this pair of operations.

Here, the measured SBP and DBP values may be stored in memory, having been obtained from a previous measurement. They may thus be historical values.

In accordance with any of the above-outlined approaches, the method may comprise communicating with the blood pressure measurement apparatus to control the pressure applicator to apply said pressure at the defined applied pressure value or within the defined applied pressure range, and to acquire the tissue pressure signal at the defined applied pressure value or within the defined applied pressure range. For example, the method may comprise controlling the pressure applicator to maintain an applied pressure at said defined pressure value or within said defined applied pressure range for a plurality of heart cycles, for example for the duration of a measurement operation. This way a continuous pulse wave signal can be derived.

The invention can also be embodied in software form. Thus, another aspect of the invention is a computer program product comprising computer program code configured, when run on a processor operatively coupled to a blood pressure measurement apparatus, to cause the processor to perform a method in accordance with any embodiment recited in this document or any claim of this application.

The invention can also be embodied in hardware.

Thus, another aspect of the invention is a processing device for use with a blood pressure measurement apparatus, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator.

The processing device may comprise an input/output; and one or more processors, operatively coupled with the input/output and configured to:

communicate with the blood pressure measurement apparatus to acquire a tissue pressure signal from the tissue pressure sensor at a defined applied pressure value or within a defined applied pressure range, and
derive a pulse wave signal from the tissue pressure signal based on extracting an AC component of the tissue pressure signal; and
wherein

(a) the defined applied pressure range is a range between 0 and 50 mmHg, for example a range between 0 and 40 mmHg;
OR
(b) the defined applied pressure value is equal to a pre-measured mean arterial pressure (MAP) value for the patient;
OR
(c) the defined applied pressure range is a range defined according to a characteristic feature or property of the tissue pressure signal which occurs in coincidence with the range, wherein the characteristic property or feature is occurrence of a maximum amplitude of the AC component of the tissue pressure signal.

Another aspect of the invention is a system, comprising: a processing device as set out above, or in accordance with any embodiment recited in this document or any claim of this application; and a blood pressure measurement apparatus operatively coupled with the processing device, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows an example blood pressure measurement apparatus;
Figs 2-4 illustrate simulated parameters for a simulated blood pressure measurement;
Fig. 5 illustrates arterial compliance and arterial cross-sectional area as a function of transmural arterial pressure;
Fig. 6 illustrates a series of comparisons performed between a simulated patient's true pulse waveform and a candidate pulse wave signal obtained from a tissue pressure sensor at a respective applied pressure value;
Fig. 7 shows a plot of a first example waveform disparity metric, M1, as a function of applied pressure;
Fig. 8 shows a plot of a second example waveform disparity metric, M2, as a function of applied pressure;
Fig. 9 shows the plot of Fig. 7 annotated to show three characteristic features of the plot for which the x-values correspond to a set of blood pressure values;
Fig. 10 shows the plot of Fig. 8 annotated to show three characteristic features of the plot for which the x-values correspond to a set of blood pressure values;
Fig. 11 shows a further example plot for metric M1;
Fig. 12 shows a further example plot for metric M2;
Fig. 13 outlines steps of any example method in accordance with one or more embodiments of the invention;
Fig. 14 outlines components of an example processing unit and system according to one or more embodiments of the invention; and
Fig. 15 shows a process for deriving a value for the defined applied pressure in accordance with one or more particular embodiments.

DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for deriving a pulse wave signal for a patient based on a tissue pressure signal measured at an artery over at least one heart/pulse cycle while an external pressure is being applied to the artery at a defined applied pressure value or range, and optionally wherein the defined pressure value is determined based on characteristics of the tissue pressure waveform.

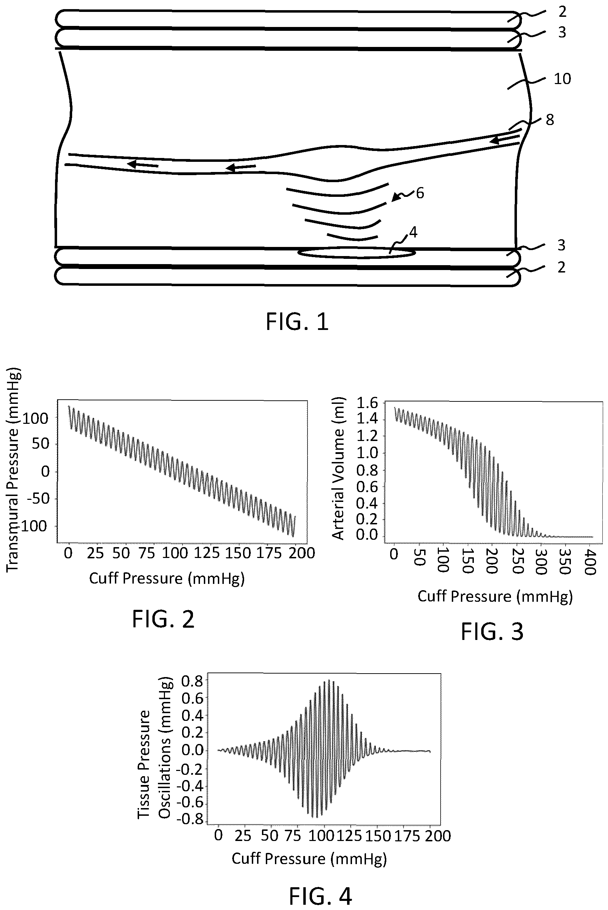

By way of background, Fig. 1 shows a schematic illustration of an example cuff-based hemodynamic parameter measurement apparatus with which embodiments of the present artefact-detection method might advantageously be applied. The apparatus can be used to detect blood pressure and/or other hemodynamic parameter measurements such as cardiac output or stroke volume.

The illustration shows the cuff applied to the upper arm 10 of a patient. An artery 8 of the patient is schematically shown. The apparatus has a design which differs from the most standard cuff-based blood pressure measurement apparatuses in that it includes a dedicated tissue pressure sensor 4 which the cuff acts to hold against the tissue of the skin when the cuff is inflated. The cuff includes a pneumatic actuator in the form of an inflatable bladder 2 for use in changing a pressure applied to the body part 10 by the cuff. The tissue pressure sensor 4 is arranged for sensing a pressure between a surface of the user's body part and the cuff. Independently of the tissue pressure sensor, one or more operational parameters of the pneumatic actuator can be sampled. For example, a pressure inside the inflatable bladder can be sensed. An actuator activity signal indicative of a pumping power level or pumping rate of a pump of the pneumatic actuator can also be sampled.

Standard blood pressure measurement cuffs use the bladder air pressure as the sole measurement signal for sensing blood pressure. By additionally utilizing a tissue pressure sensor 4, the apparatus shown in Fig. 1 can achieve superior quality results and also enables the measurement of advanced hemodynamic parameters such as stroke volume, cardiac output and fluid responsiveness in addition to blood pressure. In particular, in standard air blood pressure cuffs, dampening air is assumed to destroy > 90% of the amplitude and contour of a tissue pressure pulse wave. By contrast, by using a dedicated tissue pressure sensor, the design illustrated in Fig. 1 allows recording high-fidelity (HiFi) artery blood pressure and pulse waveform.

The tissue pressure sensor 4 may be implemented as, e.g. a fluid-filled bag which provides a conforming layer of fluid between the cuff bladder 2 and the surface of the user's body. In this way, the integrated pneumatic actuator enables hydraulic coupling of the tissue pressure sensor 4 to the upper arm tissue. When blood pulses in the artery, this results in a pressure wave 6 which can be detected by the tissue pressure sensor 4. The tissue pressure sensor 4 can be connected to a pressure transducer via a fluid-filled tube/line. The pressure transducer converts the pressure in the fluid into an electrical signal.

The operating principle is based on coupling of the pressure sensor to the body part (e.g. arm) in order to transcutaneously record tissue pressure pulse waves that result from arterial pulsation (e.g. brachial artery). In similar fashion to a conventional upper arm air blood pressure cuff, the cuff compresses the upper arm using an integrated pneumatic actuator with rising clamping pressure. However, the actual compression may be achieved via the narrowing diameter of a rigid circumferential shell 3.

In the example illustrated in Fig. 1, the cuff includes a shell portion 3, wherein the shell portion is arranged so as to be located between the pneumatic actuator 2 and the body part when the cuff is worn on the body part, and arranged to surround the body part when the cuff is worn. The tissue pressure sensor 4 is arranged so as to be positioned between the shell 3 and the body part when the device is worn on the body part. The shell structure may be relatively stiff. Consequently, the measurement accuracy can be improved, since the tissue pressure 4 is measured against a relatively stiff support, which prevents dampening of the amplitude and shape of the signal. In particular, if a tissue pressure sensor unit 4 is located at least partially between the shell portion 3 and the body part, a high accuracy of the signals measured by the tissue pressure sensor unit can be achieved. With such a configuration of the tissue pressure sensor unit, the shell structure does not absorb or attenuate the arterial pressure signal.

The shell portion 3 may include overlapping sections, and wherein the overlapping sections of the shell portion may move or slide relatively to each other, thereby reducing the diameter of the shell portion responsive to increasing applied pressure by the pneumatic actuator.

If the tissue pressure sensor uses a fluid-based sensing mechanism, as described above, then an initial calibration may be needed in which the pressure transducer, to which the fluid filled pad 4 of the pressure sensor is fluidically coupled, is exposed to atmospheric pressure prior to beginning measurement.

The design described above permits non-invasive hemodynamic monitoring and enables measurement of blood pressure, as well as cardiac output and other hemodynamic parameters.

For more expansive details about this example hemodynamic parameter measurement apparatus, reference is made to the document EP2953528 A1 which describes the cuff design in more detail.

The blood pressure measurement apparatus utilized as part of, or in combination with, some embodiments of the present invention may include some or all of the components of the hemodynamic parameter measuring system described in the document EP2953528 A1.

Embodiments of the invention might be best understood by first outlining the theoretical background underlying the inventive concepts.

To illustrate this, a simulation was performed by the inventors of an oscillometric measurement. This involved simulating arterial pressure and volume characteristics of a virtual artery for the purposes of the simulation, and these characteristics are shown in Figs. 2-3. Fig. 2 shows the simulated arterial transmural pressure (y-axis; [mmHg]), $P_T$, across the arterial wall as a function of applied pressure to the artery (x-axis; [mmHg]) used for the simulation. Fig. 3 shows the corresponding simulated arterial volume (y-axis; [ml]), $V_p$, in the virtual artery as a function of applied pressure to the artery (x-axis; [mmHg]). This was simulated using the arterial-transmural pressure - arterial-volume function as follows:

$$V_p(P_T) = 0.08 * log(0.03 * P_T + 3.3)/(1 + e^{-0.1*P_T}) * 10 \qquad (1)$$

In a real measurement, these volume oscillations are what is measured by the tissue pressure sensor 4 of the blood pressure measurement apparatus previously referenced, namely a sensor located between the inflatable cuff (or other pressure applicator) and the tissue. For example, it can be implemented by a fluid filled pad at the skin acting as a pressure sensor on top of the brachial artery. This measured tissue pressure signal has an AC component and a DC component. The AC component shows a typical pulse oscillation structure in the frequency band of arterial pulses. The DC component reflects the pressure being applied by the apparatus to the tissue, i.e. the 'applied pressure' as it is referred to in this disclosure. For present purposes, this DC component may be referred to as the tissue pressure ramp or level component, $P_{TPL}$, (also referred to as clamping pressure), and is in the frequency band which is lower than that of typical arterial pulses.

An example simulation of the AC component of the tissue pressure signal corresponding to the volume oscillations of Fig. 3 is shown in Fig. 4. The pressure ramp component $(P_{TPL})$ has been removed to leave just the AC component. In practice, extraction of the AC component of the tissue pressure signal can be done by high-pass filtering. Thus, Fig. 4 shows a simulated recording of tissue pressure signal oscillations (y-axis; [mmHg]) as a function of applied cuff pressure, $P_{ex}$ (x-axis; [mmHg]). The applied cuff pressure increases from 0 mmHg to a value above systolic pressure.

As an approximation, in the forthcoming discussion, the DC component, $P_{TPL}$, of the tissue pressure signal will be considered as equal to the applied pressure (or external pressure) $P_{ex}$ which is applied by the apparatus to the outside of the artery, and thus determining the transmural pressure $(P_T)$.

Given that the change in average applied pressure $P_{ex}$ is generally slow (~ 2mmHg / s) compared to changes in arterial pressures (max ~ 200 mmHg/s), the applied pressure may be assumed to be quasi-static (i.e. constant) over the course of any given pressure pulse.

The volume signal amplitudes are therefore determined in this case by the transmural pressure. As mentioned above, the volume changes are measured by the AC component of the tissue pressure signal.

Mathematically, the obtained pulse signals represented in the tissue pressure signal are dependent upon artery compliance $C(P_T)$, and artery compliance is non-constant as a function of the transmural pressure, $P_T$. Transmural pressure is given by the following:

$$P_T = p_A(t) - P_{ex} ,$$

where $p_A(t)$ is the time-dependent arterial blood pressure.

Thus, from the above, it will be recognized that:

The shape of a pulse wave signal, as measured by the tissue pressure sensor, will vary in dependence upon arterial compliance;
Arterial compliance varies as a function of transmural pressure; and
Transmural pressure varies as a function of the applied pressure, $P_{ex}$, applied to the artery.

Thus, it will be recognized that the waveform morphology of a pulse wave signal measured by the tissue pressure sensor will vary as a function of the applied pressure.

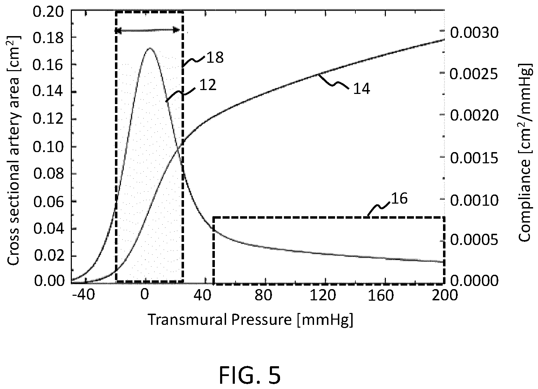

Fig. 5 shows a dependency of arterial compliance, $C(P_T)$ and arterial cross sectional area on varying transmural pressure. The x-axis shows transmural pressure ([mmHg]). The left-hand y-axis shows cross-sectional area ([cm$^2$]); the right-hand y-axis shows arterial compliance ([cm$^2$/mmHg]). Line 12 shows the arterial compliance, $C(P_T)$. Line 14 shows the cross-sectional area.

The compliance $C(P_T)$ is the derivative with respect to $P_T$ of the artery volume:

$$C(P_T) = \frac{dV_p}{dP_T} \text{ with } P_T = p_A(t) - P_{ex}. \tag{2}$$

The volume change is given by:

$$\frac{d}{dt}V_p = C(P_T) * \left(\frac{d}{dt}p_A(t) - \frac{d}{dt}P_{ex}\right). \tag{3}$$

Equation 3 shows the strong dependency of the volume change (as measured in the AC component of the tissue pressure signal) upon the characteristics of arterial compliance, C. Considering the typical measurement conditions, it holds that:

$$\frac{d}{dt}P_{ex} \ll \frac{d}{dt}p_A(t). \tag{4}$$

The impact of the artery compliance on the measured tissue pressure signal is dominated by the behavior of $C(P_T)$.

The application of the above considerations to embodiments of the present invention will now be discussed.

From the above considerations, the inventors have recognized at least two main implications.

A first main implication is that, by looking to the simulations, it is possible to identify specific ranges of transmural pressure, and thus specific ranges of applied pressure, $P_{ex}$, where the impact of arterial compliance on the morphology of a measured pulse wave signal (measured using the tissue pressure sensor) is minimal. It has thus been recognized by the inventors that by measuring the tissue pressure signal AC component within these minimal impact regions, one can obtain a pulse wave signal that most closely matches the true morphology of the patient actual pulse waveform.

A second main implication, which follows from the first, is that, by plotting the change in pulse wave morphology as a function of applied pressure, $P_{ex}$, one can identify those applied pressure values at which characteristic features in the plot occur, and infer from this the value of key blood pressure measures.

Looking to Fig. 5, reference is made to the area indicated with box 16. This is a region of the compliance, C, curve 12 at high transmural pressure values. High transmural pressure corresponds to low average applied pressure $P_{ex}$. In this region, the impact of compliance is minimal because compliance is approximately constant as a function of transmural pressure. Thus, for this region of high transmural pressure and low applied pressure, the measured tissue pressure signal over a pulse period closely corresponds to the true pulse waveform signal of the individual:

$$\frac{d}{dt}V_p = C * \frac{d}{dt}p_A(t) \quad with \: C \sim constant \tag{5}$$

Therefore, in this region of low applied pressure, the measured tissue pressure signal is proportional to the arterial pressure signal in shape.

Thus, the measured tissue pressure signal in this region of low applied pressure closely matches the pulse waveform of the patient. As will be explained below, the inventors have furthermore identified a quantitative range of applied pressure values corresponding to region 16 of Fig. 5, meaning that if the tissue pressure signal is sampled in this region, the resulting waveform can be taken as an accurate estimation of the patient's pulse waveform. Thus, this goes to the first implication mentioned above.

In contrast, for pressure levels around zero transmural pressure, the compliance function, $C(P_T)$ shows a large peak with large dependency upon transmural pressure. This region of large impact of transmural pressure is indicated by box 18. In this region, the measured tissue pressure waveform morphology is strongly impacted by the transmural pressure:

$$\frac{d}{dt} V_p = C(P_T) * \frac{d}{dt} p_A(t) \quad with\ largely\ varying\ C \qquad (6)$$

Furthermore, the compliance dependency function peaks around a transmural pressure of zero. A transmural pressure of zero means that the applied pressure, $P_{ex}$, approximately matches the arterial pressure. It follows from this that the region of applied pressures at which the measured tissue pressure waveform morphology can be expected to exhibit peak morphology variation is around a region where the applied pressure is close to at least one measure of arterial pressure. From this, it will be recognized that by plotting morphology change of measured tissue pressure signals as a function of applied pressure and looking for certain key characteristic points in the plot of morphology change (e.g. maxima, minima, inflections or zero crossings), the values of certain key blood pressure measures can be identified. This therefore goes to the second main implication mention above. This has been investigated in more detail by the inventors as will now be explained further.

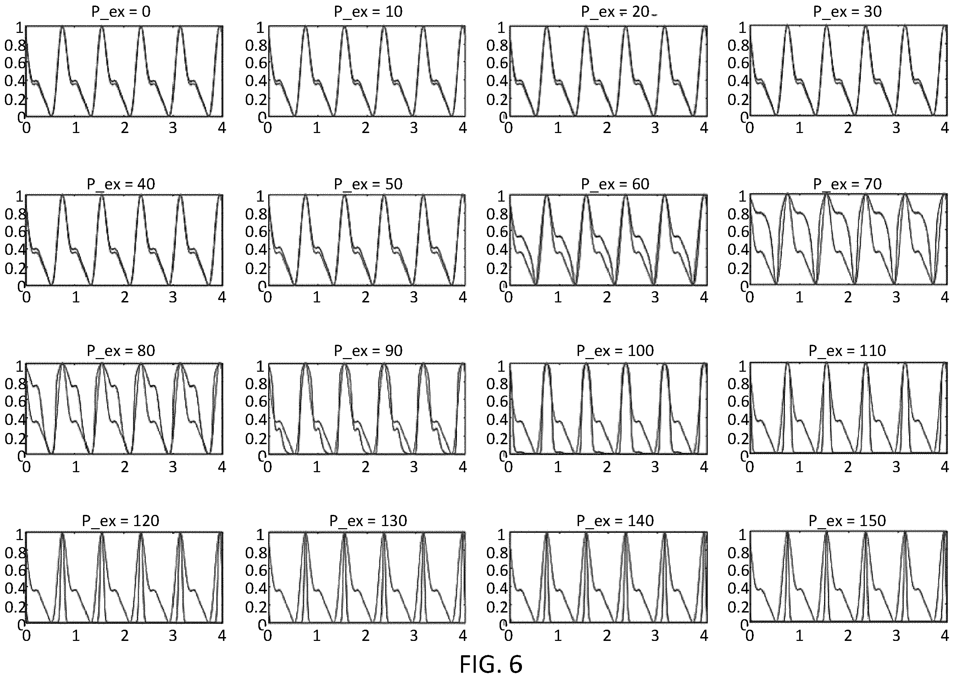

The impact of arterial blood pressure on the morphology of tissue pressure signals has been simulated and an illustrative sample of the results are visualized in Fig. 6. Each of the individual graphs of Fig. 6 shows a comparison of a respective simulated pulse waveform for simulated arterial pressure having a systolic pressure value (SBP) of 123 mmHg and a diastolic pressure value (DBP) of 90 mmHg. Thus, all of the graphs correspond to the same assumed arterial blood pressure wave. However, for each graph, a different applied pressure value ($P_{ex}$) was simulated and a corresponding tissue pressure signal simulated. The result is that each graph shows a comparison between (a) a simulated AC component of a measured tissue pressure signal over a single pulse period for one of a range of applied pressure, P_ex, values (ranging from 0-150 mmHg) and (b) a simulated true pulse waveform corresponding to arterial pressure varying between SBP = 123 mmHg and DBP = 90 mmHg. The x-axis shows time in seconds ([s]). The comparison is illustrated simply by overlaying waveform (a) atop waveform (b). This is presented in Fig. 6 for illustrative purposes only.

To enable the comparison of waveforms, the simulated signals have been normalized to the range [0 1] and scaled according to the SBP and DBP values mentioned above. The pulse period has been assumed to be constant.

To compute a quantification of each comparison, different options are possible.

By way of one example, a comparison of the scaled and normalized pulse waveforms (per pulse k) can be achieved by computing a waveform disparity metric (M 1) as an integral of a difference between the signals over a pulse wave period, $T_k = T_{k\_end} - T_{k\_start}$, where $T_k$ is the pulse wave period for pulse $k$, $T_{k\_start}$ is the time of the start of the pulse $k$ and $T_{k\_end}$ is the time at the end of the pulse k. By way of a further example, a comparison of the scaled and normalized pulse waveforms (per pulse k) can be achieved by computing a waveform disparity metric (M2) equal to an integral of a square of the difference between the waveforms, again over a pulse wave period, $T_k = T_{k\_end} - T_{k\_start}$,

These can be quantified as follows:

$$M1 = \frac{1}{K} \sum_k \frac{1}{T_{k\_end} - T_{k\_start}} \int_{T_{k\_start}}^{T_{k\_end}} \left( s_1(t) - s_2(t - T_{k\_start}) \right) dt \qquad (7a)$$

or

$$M1 = \frac{1}{\sum_k (T_{k\_end} - T_{k\_start})} \sum_k \int_{T_{k\_start}}^{T_{k\_end}} \left( s_1(t) - s_2(t - T_{k\_start}) \right) dt \qquad (7b)$$

and

$$M2 = \frac{1}{K} \sum_k \frac{1}{T_{k\_end} - T_{k\_start}} \int_{T_{k\_start}}^{T_{k\_end}} \left( s_1(t) - s_2(t - T_{k\_start}) \right)^2 dt \qquad (8a)$$

or

$$M2 = \frac{1}{\sum_k (T_{k\_end} - T_{k\_start})} \sum_k \int_{T_{k\_start}}^{T_{k\_end}} \left( s_1(t) - s_2(t - T_{k\_start}) \right)^2 dt \qquad (8b)$$

For the performed simulation, for each comparison, $s_1$ corresponds to the simulated tissue pressure signal at one of the applied pressures, and $s_2$ corresponds to the simulated true pulse waveform.

In the above expressions, K is the total number of pulses used to determine the disparity metric. For consecutive pulses it typically holds that $T_{k+1\_start} = T_{k\_end}$, i.e., the end of the preceding pulse is the start of the next pulse. This holds in case of no missing pulses due to e.g. misdetections. The simulated true pulse wave signal $s_2(t)$ is considered to run from 0 to $T_k = T_{k\_end} - T_{k\_start}$. In a simplest case, each disparity metrics M1 and M2 may be computed for a single individual pulse only, and thus the summations in Equations (7a), (7b) and (8a), (8b) would contain only a single element. In this case, Equations (7a) and (b) would reduce to the same expression and Equations (8a) and (8b) would reduce to the same expression. Furthermore, in case the duration of each pulse has been scaled to a same duration period, e.g. 1 s, the difference $T_{k\_end} - T_{k\_start}$ is equal to that scaled duration period, e.g. 1 s, for each individual pulse k.

It is noted that, as a variation on the above, an M1 metric could be determined from the absolute difference between $s_1(t)$ and $s_2(t)$, i.e., by integrating $|s_1(t) - s_2(t - T_{k\_start})|$ instead of $(s_1(t)) - s_2(t - T_{k\_start}))$.

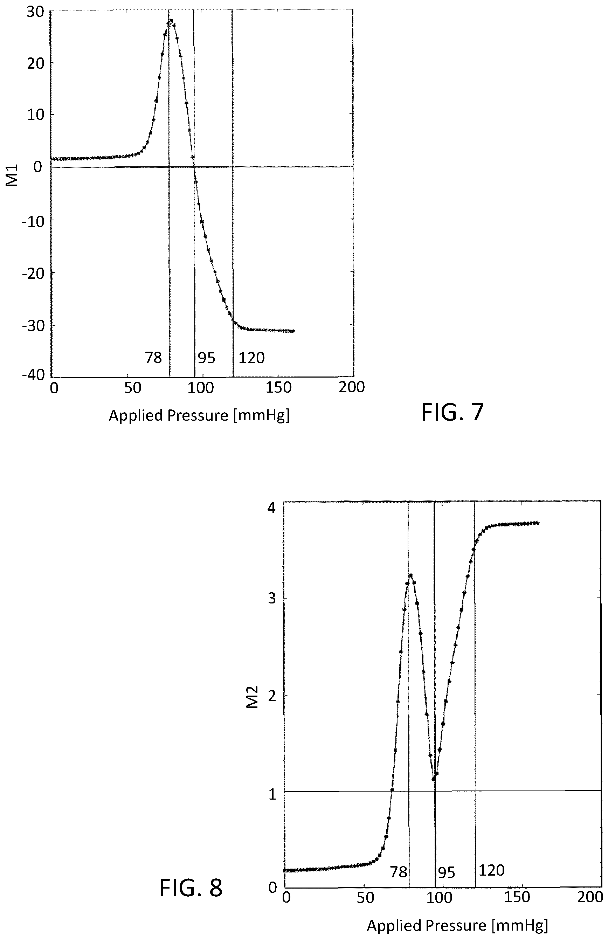

Fig. 7 shows an example plot of the first waveform disparity metric M1 (y-axis) as a function of applied pressure (x-axis; [mmHg]), where the summations in M1 contain only a single element: i.e., K=1 and the disparity is determined at each applied pressure value for a single individual pulse only. In this case, the two expressions given for M1 above reduce to the same single expression $\dfrac{1}{T_{k\_end} - T_{k\_start}} \int_{T_{k\_start}}^{T_{k\_end}} \left( s_1(t) - s_2\left(t - T_{k\_start}\right) \right) dt$ .

Fig. 8 shows a plot of the second waveform disparity M2 (y-axis) as a function of applied pressure (x-axis; [mmHg]), where the summations in M2 contain only a single element: i.e., K=1 and the disparity is determined at each applied pressure value for a single individual pulse only. In this case, the two expressions given for M2 above reduce to the same single expression $\dfrac{1}{T_{k\_end} - T_{k\_start}} \int_{T_{k\_start}}^{T_{k\_end}} \left( s_1(t) - s_2\left(t - T_{k\_start}\right) \right)^2 dt$

For Fig 7, differences have directly been determined between the pulses. For Fig. 8, differences have been determined between pulses which had been normalized and scaled for more exact comparison. Hence the difference in range of the vertical axes of Fig. 7 and Fig. 8.

Indicated on each graph are indicative vertical lines which show the locations along each plot where the applied pressure (x-axis) was equal to the stimulated diastolic blood pressure (DBP = 78 mmHg), mean arterial blood pressure (MAP = 95 mmHg) and systolic blood pressure (SBP = 120 mmHg) of the simulated artery.

It can be seen that on each graph, the points where the applied cuff pressure (x-axis) matched these three respective values of blood pressure correspond to characteristic features of the respective plots for M1 (Fig. 7) and M2 (Fig. 8).

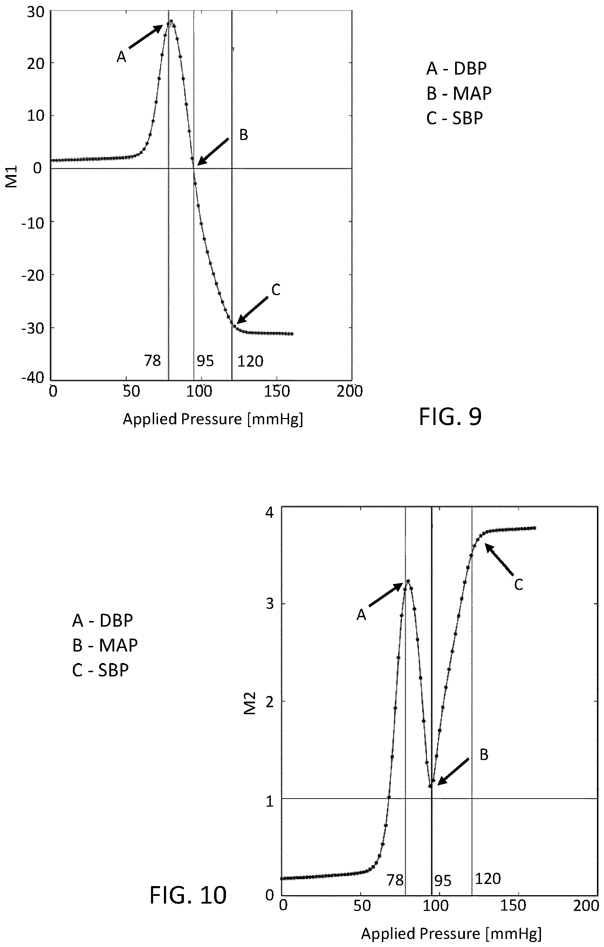

This is illustrated more explicitly in Fig. 9 and Fig. 10 which respectively show a first (A), second (B) and third (C) feature of each plot, where the first feature in each plot coincides with an applied pressure value which equals the diastolic blood pressure (DBP), the second feature (B) of each plot coincides with an applied pressure value which equals the mean arterial blood pressure (MAP) and the third feature (C) of each plot coincides with an applied pressure value which is equal to the systolic blood pressure (SBP). The features follow one Ir in sequence.

In the plot for M1: the plot feature (A) coinciding with applied pressure equal to DBP is a first local maximum point of the plot; the plot feature (B) coinciding with applied pressure equal to MAP is a zero-crossing point of the plot; and the plot feature (C) coinciding with applied pressure equal to SBP is a first inflection point of the plot following the zero crossing point.

In the plot for M2: the plot feature (A) coinciding with applied pressure equal to DBP is a first local maximum point of the plot; the plot feature (B) coinciding with applied pressure equal to MAP is a first local minimum point of the plot; and the plot feature (C) coinciding with applied pressure equal to SBP is a final inflection point of the plot, or a next inflection point following said first local minimum point (B).

A further observation apparent from each plot is that, for applied pressure values below about 40-50 mmHg, each of the disparity metrics M1, M2 have only a very small, roughly constant value. From this it can be concluded that a sample pulse wave signal measured with the tissue pressure sensor within this applied pressure range can be expected to closely match the waveform morphology of the true pulse wave signal for the patient (in view of the fact that the plots of M1 and M2 in Fig. 9 and Fig. 10 represent disparity in the waveform morphology of a respective pulse waveform measured with the tissue pressure sensor at a given applied pressure with the simulated true pulse waveform for the patient).

From the above, it has been recognized by the inventors that these technical observations could be applied in reverse in order to:

(a) obtain a pulse waveform for a patient which closely matches the true pulse waveform morphology (i.e. an "A-line like" waveform), based on acquiring a sample pulse waveform with the tissue pressure sensor within an applied pressure range for which the waveform disparity metric is known to be low (e.g. in the range 0-40 mmHg or 0-50 mmHg, or around the point B corresponding to mean arterial pressure).

(b) derive unknown values of DBP, MAP and SBP based on generating a plot of a waveform disparity metric such as M1 or M2 from an acquired sequence of sample pulse wave signals measured with a tissue pressure sensor at a sequence of different applied pressure values, each compared against a reference pulse wave signal acquired at an applied pressure value where waveform disparity is known to be low (e.g. in the range 0-40 mmHg or 0-50 mmHg or around the point B corresponding to mean arterial pressure).

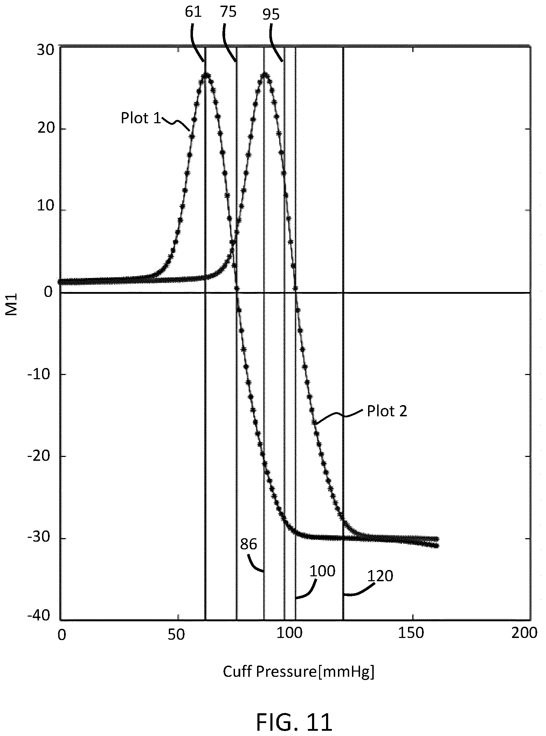

This was tested and confirmed with a further plot for M1, labelled Plot 1 in Fig. 11. Plot 1 was generated for simulated blood pressure values: DBP=61 mmHg; MBP=75 mmHg, SBP=95 mmHg. Plot 2 in Fig. 11 shows, for comparison, a plot generated for simulated blood pressure values: DBP=86 mmHg, MBP=100 mmHg, SBP=120 mmHg. For the lower blood pressure of Plot 1, the curve shifts to the left. It can be seen that, for Plot 1 and Plot 2, the applied pressure values coincide with the simulated DBP, MAP and SBP values at the same characteristic plot features A, B and C labelled in Fig. 9.

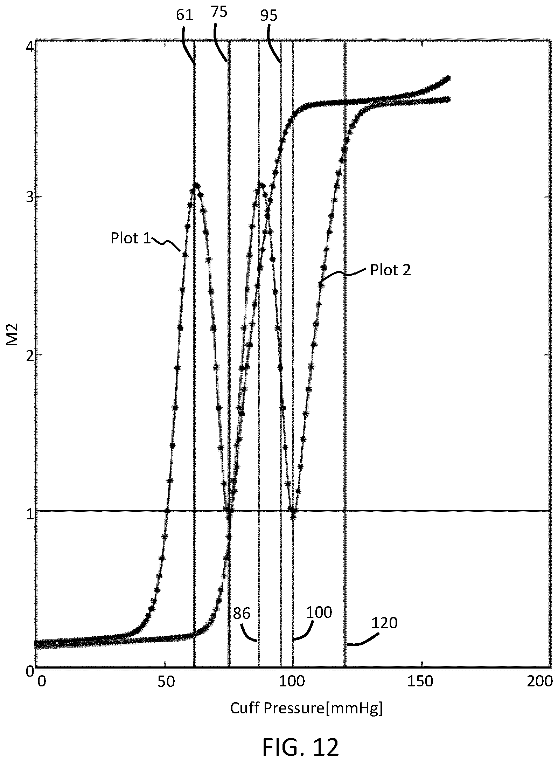

Fig. 12 shows a further plot for M2, labelled Plot 1 in Fig. 12. Plot 1 was generated for simulated blood pressure values: DBP=61 mmHg; MBP=75 mmHg, SBP=95 mmHg Plot 2 in Fig. 12 shows, for comparison, a plot generated for simulated blood pressure values: DBP=86 mmHg, MBP=100 mmHg, SBP=120 mmHg. For the lower blood pressure of Plot 1, the curve shifts to the left. It can be seen that, for Plot 1 and Plot 2, the applied pressure values coincide with the simulated DBP, MAP and SBP values at the same characteristic plot features A, B and C labelled in Fig. 10.

It is the proposal of the inventors to apply these considerations in practice to enable inference of an A-line like pulse waveform measurement for the patient based on acquiring a tissue pressure signal waveform while an external pressure is being applied to relevant artery within a defined range of applied pressures known to coincide with low waveform disparity with respect to the patient's true pulse waveform.

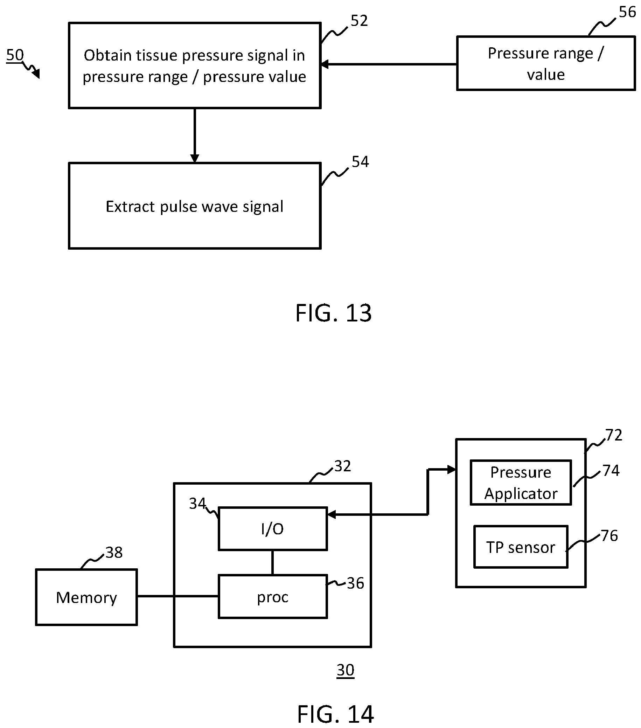

Fig. 13 outlines in block diagram form steps of an example computer-implemented method 50 according to one or more embodiments. The steps will be recited in summary, before being explained further in the form of example embodiments.

Provided is a method 50 for use in blood pressure measurement by a blood pressure measurement apparatus, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator.

The method comprises acquiring 52 a tissue pressure signal from the tissue pressure sensor within a defined applied pressure range 56. The tissue pressure signal should be acquired over at least one heart cycle, and preferably a plurality of heart cycles.

The method further comprises deriving 54 a pulse wave signal from the tissue pressure signal based on extracting an AC component of the tissue pressure signal (over at least one heart cycle).

With regards to the defined applied pressure range 56, this may be defined quantitatively in advance, or it might be determined dynamically during the measurement process.

In some embodiments, (a) the defined applied pressure range is a range between 0 and 50 mmHg (= 0 to 6666 Pa), for example between 0 and 40 mmHg (= 0 to 5332 Pa), for example 10 to 40 mmHg (=1333 to 5332 Pa).

In some embodiments (b) the defined applied pressure range is a single-value range equal to a pre-measured mean arterial pressure (MAP) value for the patient.

In some embodiments, (c) the defined applied pressure range is a range defined according to a characteristic feature or property of the tissue pressure signal which occurs in coincidence with the range, wherein the characteristic property or feature is occurrence of a maximum amplitude of the AC component of the tissue pressure signal.

The method preferably may further comprise generating a data output indicative of the one or more arterial blood pressure measures.

As noted above, the method can also be embodied in hardware form, for example in the form of a processing unit which is configured to carry out a method in accordance with any example or embodiment described in this document, or in accordance with any claim of this application.

To further aid understanding, Fig. 14 presents a schematic representation of an example processing unit 32 configured to execute a method in accordance with one or more embodiments of the invention. The processing unit is shown in the context of a system 30 which comprises the processing unit. The processing unit alone represents an aspect of the invention. The system 30 is another aspect of the invention. The provided system does not have to comprise all of the illustrated hardware components; it may just comprise a subset of them.

The processing unit 32 comprises one or more processors 36 configured to perform a method in accordance with that outlined above, or in accordance with any embodiment described in this document or any claim of this application. In the illustrated example, the processing unit further comprises an input/output 34.

In the illustrated example of Fig. 14, the system 30 further comprises a blood pressure measurement apparatus 72. The blood pressure measurement apparatus 72 may comprise a pressure applicator 74 for use in applying a variable pressure to the tissue of a body part above a blood vessel, and further includes a tissue pressure sensor 76 arranged for sensing a pressure between a surface of the user's body part above the blood vessel and the pressure applicator. The blood pressure measurement apparatus may comprise a cuff for being worn by the patient, and wherein the pressure applicator 74 takes the form of a pneumatic actuator comprising an inflatable bladder, and wherein a varying inflation level of the bladder varies the applied pressure.

In some embodiments, the system 30 further comprises a user interface (not shown) for displaying the derived pulse wave signal.

The input/output 34 may be adapted for receiving the previously mentioned series of tissue pressure signal, and optionally for outwardly communicating the data output indicative of the one or more arterial blood pressure measures. The tissue pressure signals might alternatively be received from a datastore, or from an intermediary communication device, such as a hub server or a network node.

For example, the input/output 34 may be adapted for wired or wireless connection to one or more external units. These might include the blood pressure measurement apparatus.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

The blood pressure measurement apparatus may optionally take the form of the blood pressure measurement device detailed in EP2953528 A1 and described earlier in this document.

As mentioned previously, the invention can also be embodied in software form. Thus another aspect of the invention is a computer program product comprising code means configured, when run on a processor, to cause the processor to perform a method in accordance with any example or embodiment of the invention described in this document, or in accordance with any claim of this patent application.

Embodiments of this invention are based on the observations derived from the simulations outlined in detail above.

With regards to option (a), as was demonstrated above with reference to Figs. 7-10, for applied pressure values below about 40-50 mmHg, each of the waveform disparity metrics M1, M2 have only a very small, roughly constant value. From this it can be concluded that a sample pulse wave signal measured with the tissue pressure sensor within this applied pressure range can be expected to closely match the waveform morphology of the true pulse wave signal for the patient. By way of reminder, each of the waveform disparity metrics (M1, M2) represent a comparison between a waveform morphology of a sample pulse wave signal (scaled and normalized AC component of a sampled tissue pressure signal) acquired at a respective applied pressure value (x-axis in Figs 7-10) and the patient's true pulse waveform.

With regards to option (b), as was also demonstrated above with reference to Figs. 7-10, next to the range of about 0-50 or 0-40 mmHg, the applied pressure range resulting in the next lowest waveform disparity is the applied pressure at around a value equal to the mean arterial pressure (MAP) for the patient, that is: point B in Fig. 9 and Fig. 10. As apparent from Fig. 9, at an applied pressure equal to MAP (point B), M1 has a value of zero, and as apparent from Fig. 10, at an applied pressure equal to MAP, M2 has a value which is at local minimum.

With regards to option (c), this is in fact an alternative way to target the same pressure value or range as in option (b). In particular, a maximum of the AC component of the tissue pressure signal is expected to coincide with an applied pressure value equal to the MAP. This is because the amplitude of the AC component of the tissue pressure signal can be expected to be at a maximum when transmural pressure is at a minimum. As illustrated in Fig. 5, when transmural pressure is at a minimum, the arterial compliance is at a maximum. The amplitude of the AC component of the tissue pressure signal can be expected to scale with arterial compliance. Thus, when the AC component of the tissue pressure signal is at a maximum, transmural pressure is approximately zero. Since transmural pressure = arterial pressure - applied pressure, when transmural pressure is zero on average, the applied pressure equals the mean arterial pressure (MAP). As explained above, at an applied pressure approximately equal to the MAP, M1 has a value of zero and M2 is at a local minimum.

With regards to any of the above-outlined options, preferably scaling is further applied to the extracted AC component of the tissue pressure signal as part of deriving the final pulse wave signal. The scaling comprises scaling the extracted AC component of the tissue pressure signal, wherein the scaling comprises maintaining a waveform shape/morphology, but increasing the scale of the waveform such that a maximum point of the waveform matches an estimated or pre-measured SBP value, and the minimum point matches an estimated or pre-measured DBP value. The SBP and DBP values used for the scaling may be previously measured values recalled from a memory or datastore, or they might be estimated based on standard values for the patient based on a demographic category of the patient, or might be estimated from other physiological measurements such as a PPG signal. Even without the scaling, the obtained tissue pressure signal AC component still provides valuable clinical information because its shape accurately reflects the morphology of the patient's true pulse waveform.

With regards to any of the above-outlined options, accuracy can be improved by generating the pulse wave signal based on an average over multiple tissue pressure signal pulses all acquired at a same applied pressure value. In other words, it is generated by sampling the tissue pressure signal over a plurality of heart cycles at a constant applied pressure value within the defined applied pressure range, and computing an average tissue pressure signal over the multiple heart cycles as the final tissue pressure signal. From this, this final pulse wave signal is obtained. This increases signal-to-noise ratio. For example, a pulse segmentation algorithm may be used to extract a pulse signal per heart phase period. Optionally, scaling in the time domain may be applied to the plurality of obtained pulse signals (to compensate for varying heart rates). Optionally, before averaging, the amplitude and the period of the pulse could be normalized in order to obtain a cleaner averaged pulse. Averaging of the pulses might then be achieved for example by first aligning or registering all of the pulse signals in time based on alignment of pre-defined fiducial points in the signal, e.g. a specific time point in the pulse phase. The final averaged signal may then be obtained averaging all time-aligned pulses.

Additionally or alternatively, with regards to any of the above-outlined options, preferably normalization is applied to the AC component of the tissue pressure signal as part of deriving the final pulse wave signal.

The skilled person will recognise how such normalisation may be performed. By way of example, the tissue pressure signal could be processed as follows in order to extract individual pulse signals and normalise these in duration and amplitude. First, the tissue pressure signal from the tissue pressure sensor may be processed to identify the start and end points of individual pulses in the signal, via a pulse detection algorithm. Pulse detection algorithms are well known in the art. A next step may be to scale a duration of each of the pulses to a same duration, e.g. 1 second. Various algorithms are also known in the art for achieving this, such as dynamic time warping. A next step may be to scale the AC component of the signal to e.g. unity. For example, each pulse signal may be divided by a maximum value, to set the systolic level to 1. Another option may be to normalize each pulse signal such that the peak-to-valley pulse height is equal to unity.

With regards to option (c), as has been discussed, this option involves dynamically determining what value of applied pressure to use while acquiring the tissue pressure signal. In particular, it is proposed to use an applied pressure value which coincides with a maximum (or at least a local maximum) AC amplitude of the tissue pressure signal.

In some embodiments, the applied pressure value which coincides with this characteristic feature of the tissue pressure signal might be determined in advance, and wherein the method simply recalls the appropriate applied pressure value from a memory. Nonetheless, it would be easily possible to test whether the applied pressure value did indeed coincide with the required characteristic of the tissue pressure signal by simply scanning through different applied pressure values and identifying the applied pressure value at which the tissue pressure signal amplitude reaches a local maximum.

In some embodiments, the applied pressure value which coincides with this characteristic feature of the tissue pressure signal might be determined as part of the claimed method itself, either as an initial calibration procedure, before measurement properly begins, or as part of the measurement process itself.

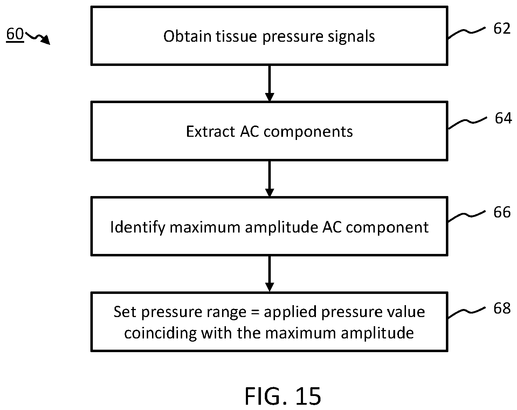

To illustrate one approach, Fig. 15 outlines steps of an example process 60 for determining the defined range of applied pressures within which the tissue pressure signal is to be acquired.

The process comprises obtaining 62, using the blood pressure measurement apparatus 72, a series of tissue pressure signals, each acquired at a different applied pressure value across a sequence of incrementally increasing or decreasing applied pressure values.

The process comprises extracting 64 the respective AC component from each applied pressure value to obtain a series of AC-extracted tissue pressure signals. The process further comprises identifying 66 from the series of AC-extracted tissue pressure signals, the one with the highest amplitude. Alternatively, or additionally, it might comprise plotting the AC amplitudes of the series of signals as a function of applied pressure and identifying a first local maximum in the plot, e.g. after applying appropriate interpolation, and identifying the applied pressure value coinciding with this first local maximum, e.g. of the interpolated plot, as the defined applied pressure value to use for acquiring the pulse wave signal. The process 60 further comprises setting 68 the defined applied pressure range equal to the applied pressure coinciding with the identified maximum (or local maximum) AC amplitude tissue pressure signal.

In other words, the defined applied pressure range is identified from analysis of the AC components of the acquired series of tissue pressure signals.

The AC component of each tissue pressure signal might be acquired in real time as each tissue pressure signal is acquired, or all of the tissue pressure signals might be acquired and then the AC components are extracted in a batch process at the end. The real-time approach has the advantage that, optionally, the applied pressure sequence could be ended as soon as a local maximum in the AC amplitude of the tissue pressure signals has been identified. This reduces the total time for the calibration operation.

In some embodiments, the method comprises controlling the blood pressure measurement apparatus 72 to acquire the sequence of tissue pressure signals. In some embodiments, the method comprises communicating with the blood pressure measurement apparatus to control a tissue application pressure cycle of the blood pressure measurement apparatus in which tissue application pressure is incrementally increased or decreased across an application pressure range to realize a sequence of different applied pressures which include said defined range of applied pressures.

In accordance with any of the above-outlined approaches, the method may comprise communicating with the blood pressure measurement apparatus 72 to control the pressure applicator to apply said pressure within the defined applied pressure range, and to acquire the tissue pressure signal within the defined applied pressure range. For example, the method may comprise controlling the pressure applicator to maintain an applied pressure within said defined applied pressure range for a plurality of heart cycles, for example for the duration of a measurement operation. This way a continuous pulse wave signal can be derived.

In accordance with any of the above-outlined approaches, the method may comprise communicating with a user interface to display the acquired pulse wave signal. It may be displayed on a screen of the user interface in graphical form, e.g. in the form of a waveform. It may be labelled with an on-screen annotation indicating that the signal is a pulse wave signal for the patient.

The pulse wave signal may be recurrently re-measured by repeating the steps of the method 50 of Fig. 13 on a loop. The method may comprise setting the applied pressure of the blood pressure measurement apparatus 72 to a value within the defined range and then holding that pressure constant while the pulse wave signal is continuously or recurrently acquired and displayed on the user interface display.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A method for obtaining a pulse wave signal for a patient, the method employing use of a blood pressure measurement apparatus, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator:
   the method comprising:

acquiring a tissue pressure signal from the tissue pressure sensor at a defined applied pressure value or within a defined applied pressure range, and

deriving a pulse wave signal from the tissue pressure signal based on extracting an AC component of the tissue pressure signal; and

wherein

(a) the defined applied pressure range is a range between 0 and 50 mmHg (= 0 to 6666 Pa).;
OR
(b) the defined applied pressure value is equal to a pre-measured mean arterial pressure (MAP) value for the patient;
OR
(c) the defined applied pressure range is a range defined according to a characteristic feature or property of the tissue pressure signal which occurs in coincidence with the range, wherein the characteristic property or feature is occurrence of a maximum amplitude of the AC component of the tissue pressure signal.

2. The method of claim 1, further comprising generating a data output representing the derived pulse wave signal.

3. The method of claim 2, further comprising communicating the data output to a user interface for display to a user.

4. The method of any of claims 1-3,

wherein the method comprises feature (c),
wherein the method comprises obtaining using the blood pressure measurement apparatus a series of tissue pressure signals, each acquired at a different applied pressure values across a sequence of incrementally increasing or decreasing applied pressure value; and
wherein the applied pressure value coinciding with the maximum amplitude of the AC component of the tissue pressure signal is identified from analysis of the AC components of the series of tissue pressure signals.

5. The method of any of claims 1-4, wherein acquiring the tissue pressure signal from the tissue pressure sensor at the defined applied pressure value or within the defined applied pressure range comprises sampling a tissue pressure signal over a plurality of heart cycles at a constant applied pressure value, and computing an average tissue pressure signal over the plurality of heart cycles as the acquired tissue pressure signal.

6. The method of any of claims 1-5, wherein the method further comprises scaling the extracted AC component of the tissue pressure signal, wherein the scaling comprises maintaining a waveform shape/morphology, but increasing the scale of the waveform such that a maximum point of the waveform matches a pre-measured SBP value, and the minimum point matches a pre-measured DBP value.

7. The method of any of claims 1-6, where the pre-defined applied pressure range is a range between 10-40 mmHg (=1333 to 5332 Pa).

8. The method of any of claims 1-7, wherein the method comprises communicating with the blood pressure measurement apparatus to control the pressure applicator to apply said pressure at the defined applied pressure value or within the defined applied pressure range, and to acquire the tissue pressure signal at the defined pressure value or within the defined applied pressure range.

9. The method of any of claims 1-8, wherein the method comprises communicating with the blood pressure measurement apparatus to control a tissue application pressure cycle of the blood pressure measurement apparatus in which tissue application pressure is incrementally increased or decreased across an application pressure range to realize a sequence of different applied pressures which include said defined range of applied pressures or said defined applied pressure value.

10. The method of any of claims 1-9, wherein the blood pressure measurement apparatus includes a cuff for wrapping around a part of the body of a user, and wherein the cuff incorporates the pressure applicator, and wherein the pressure applicator is a pneumatic actuator in the form of an inflatable bladder.

11. A computer program product comprising computer program code configured, when run on a processor operatively coupled to a blood pressure measurement apparatus, to cause the processor to perform a method in accordance with any of claims 1-10.

12. A processing device for use with a blood pressure measurement apparatus, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator,

the processing device comprising:
an input/output; and
one or more processors, operatively coupled with the input/output and configured to:

communicate with the blood pressure measurement apparatus to acquire a tissue pressure signal from the tissue pressure sensor at a pre-defined applied pressure value or within a pre-defined applied pressure range, and
derive a pulse wave signal from the tissue pressure signal based on extracting an AC component of the tissue pressure signal; and
wherein

(a) the defined applied pressure range is a range between 0 and 50 mmHg;
OR
(b) the defined applied pressure value is equal to a pre-measured mean arterial pressure (MAP) value for the patient;
OR
(c) the defined applied pressure range is a range defined according to a characteristic feature or property of the tissue pressure signal which occurs in coincidence with the range, wherein the characteristic property or feature is occurrence of a maximum amplitude of the AC component of the tissue pressure signal.

13. A system, comprising:

a processing device as claimed in claim 12; and
a blood pressure measurement apparatus operatively coupled with the processing device, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator.

FIG. 1

FIG. 2

FIG. 3

FIG. 4

FIG. 5

FIG. 6

EP 4 400 040 A1

FIG. 7

FIG. 8

A - DBP
B - MAP
C - SBP

**FIG. 9**

A - DBP
B - MAP
C - SBP

**FIG. 10**

FIG. 11

FIG. 12

FIG. 13

FIG. 14

60

| Obtain tissue pressure signals | 62 |

↓

| Extract AC components | 64 |

↓

| Identify maximum amplitude AC component | 66 |

↓

| Set pressure range = applied pressure value coinciding with the maximum amplitude | 68 |

## FIG. 15

Europäisches
Patentamt

European
Patent Office

Office européen
des brevets

# EUROPEAN SEARCH REPORT

Application Number

EP 23 15 1043

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/298188 A1 (DANA ALEXANDRA [IL] ET AL) 3 October 2019 (2019-10-03) * paragraphs [0047], [0059] – [0097]; claims; figures * ----- | 1-13 | INV. A61B5/022 A61B5/00 |
| X | US 2017/360313 A1 (BAEK DAVID BOETTCHER [US] ET AL) 21 December 2017 (2017-12-21) * paragraphs [0055] – [0096]; claims; figures * ----- | 1-13 | |

TECHNICAL FIELDS SEARCHED (IPC)

A61B

The present search report has been drawn up for all claims

| Place of search | Date of completion of the search | Examiner |
|---|---|---|
| The Hague | 25 June 2023 | Crisan, Carmen-Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 1043

This annex lists the patent family members relating to the patent documents cited in the above-mentioned European search report.
The members are as contained in the European Patent Office EDP file on
The European Patent Office is in no way liable for these particulars which are merely given for the purpose of information.

25-06-2023

| Patent document cited in search report | | Publication date | Patent family member(s) | | Publication date |
|---|---|---|---|---|---|
| US 2019298188 | A1 | 03-10-2019 | CN | 110301906 A | 08-10-2019 |
| | | | DE | 102019104568 A1 | 02-10-2019 |
| | | | KR | 20190113543 A | 08-10-2019 |
| | | | US | 2019298188 A1 | 03-10-2019 |
| US 2017360313 | A1 | 21-12-2017 | CN | 109310349 A | 05-02-2019 |
| | | | US | 2017360313 A1 | 21-12-2017 |
| | | | WO | 2017222700 A1 | 28-12-2017 |

EPO FORM P0459

For more details about this annex : see Official Journal of the European Patent Office, No. 12/82

**EP 4 400 040 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2953528 A1
- US 20150320364 A1